# EUROPEAN PATENT APPLICATION

(11) **EP 2 422 725 A2**
(43) Date of publication of application: **29.02.2012**
(21) Application number: 11250736.3
(22) Date of filing: 24.08.2011
(51) Int. Cl.: A61B 17/34

(54) **Thoracic access port**

(30) Priority: 25.08.2010 US 376726 P; 13.01.2011 US 5611; 23.06.2011 US 166883
(71) Applicant: Tyco Healthcare Group LP, New Haven, CT 06511 (US)
(72) Inventor: O'Prey, Cormac, Bishops Stortford Hertfordshire CM23 4HH (GB); Ansell, Iain, Newmarket Suffolk CB8 7DZ (GB); Chan, Wai Ting, Cambridge CB1 2RU (GB); Middlemiss Haig, Fiona, Histon Cambridge CB24 9JG (GB); Scott, Valerie Anne, Cambridge CB4 2DB (GB); Clark, Charlotte Adele, Cambridge CB1 3EG (GB)
(74) Representative: Soames, Candida Jane

(57) **Abstract**

A surgical access assembly having a body including a leading end, a trailing end, and first and second body members extending between the leading and trailing ends. The leading end, trailing end and first and second body members define a passageway therethrough for receipt of surgical instrumentation. First and second flexible wing members extend proximally from the body. A flexible member is attached to the body and extends proximally therefrom.

## Description

### BACKGROUND

This application claims priority from provisional application serial no. 61/376,726, filed August 25, 2010, and is a continuation in part of application serial no. 13/005,611, filed January 13, 2011, which claims priority from provisional application serial no. 61/304,083, filed February 12, 2010. The entire contents of which are incorporated herein by reference.

### 1. Technical Field

The present disclosure relates generally to devices and techniques for performing surgical procedures. More particularly, the present disclosure relates to access devices for minimally invasive surgery.

### 2. Background of the Related Art

In an effort to reduce trauma and recovery time, many surgical procedures are performed through small openings in the skin, such as an incision or a natural body orifice. For example, these procedures include laparoscopic procedures, which are generally performed within the confines of a patient's abdomen, and thoracic procedures, which are generally performed within a patient's chest cavity.

Specific surgical instruments have been developed for use during such minimally invasive surgical procedures. These surgical instruments typically include an elongated shaft with operative structure positioned at a distal end thereof, such as graspers, clip appliers, specimen retrieval bags, etc.

During minimally invasive procedures, the clinician creates an opening in the patient's body wall, oftentimes by using an obturator or trocar, and thereafter positions an access assembly within the opening. The access assembly includes a passageway extending therethrough to receive one or more of the above-mentioned surgical instruments for positioning within the internal work site, e.g. the body cavity.

During minimally invasive thoracic procedures, an access assembly is generally inserted into a space located between the patient's adjacent ribs that is known as the intercostal space, and then surgical instruments can be inserted into the internal work site through the passageway in the access assembly.

In the interests of facilitating visualization, the introduction of certain surgical instruments, and/or the removal of tissue specimens during minimally invasive thoracic procedures, it may be desirable to spread tissue adjacent the ribs defining the intercostal space. Additionally, during these procedures, firm, reliable placement of the access assembly is desirable to allow the access assembly to withstand forces that are applied during manipulation of the instrument(s) inserted therethrough. However, reducing patient trauma during the procedure, discomfort during recovery, and the overall recovery time remain issues of importance. Thus, there exists a need for thoracic access ports which minimize post operative patient pain while enabling atraumatic retraction of tissue and which do not restrict access to the body cavity, as well as facilitates removal of tissue specimens from the body cavity.

### SUMMARY

In accordance with the present disclosure, there is disclosed a surgical access assembly for positioning within an opening in tissue. The surgical access assembly generally includes a body having a leading end, a trailing end and first and second body members extending between the leading end and the trailing end. The leading end, trailing end and first and second body members define a passageway therethrough. First and second flexible wing members extend proximally from the body. A flexible member is affixed to the body, extending proximally therefrom, and surrounding the passageway.

In some embodiments, the flexible member is a flexible membrane, and a distal end of the flexible membrane is affixed to a membrane bonding surface on the body. In some embodiments, at least a portion of the flexible membrane extends through the passageway.

At least one of the leading end and trailing end can have a ribbon port formed therethrough.

In some embodiments, the flexible wing members each have a free end, the free end of each wing member movable from a first position to a second position where the free ends are spaced further apart. Each of the first and second flexible wing members can have a concave outward facing surface for engagement with the tissue adjacent the ribs of a patient.

In some embodiments, the first and second flexible wing members extend into the passageway.

In some embodiments, each of the first and second flexible wing members decreases in thickness from the first and second body members to first and second free ends of the first and second flexible wings.

In some embodiments, the first and second wing members are on opposing sides of the passageway. The wing members in some embodiments are oriented along the length of the body.

In some embodiments, the first and second body members include first and second central fold lines formed in the first and second body members. The first and second central fold lines can be formed in upper surfaces of the first and second body members and the second and third central fold lines can be formed in under surfaces of the first and second body members. The first flexible wing member can be connected to the first body member along a first wing fold and the first central fold line can bisect the first wing fold and the second flexible wing member can be connected to the second body member along a second wing fold and the second central fold line can bisect the second wing fold.

### BRIEF DESCRIPTION OF THE DRAWINGS

Various embodiments of the subject access port are described herein with reference to the drawings wherein:

FIG. 1 is a side view of an access port of one embodiment according to the present disclosure shown being inserted into an incision in tissue;

FIG. 2A is a bottom view of the access port of FIG. 1 being rotated into position within the incision in tissue;

FIG. 2B is a bottom view of the access port of FIG. 1 in position for movement between an approximated and an open position;

FIG. 3 is a side, cross-sectional view of the access port of FIG. 1 disposed in the open position;

FIG. 4 is a bottom, perspective view of the access port of FIG. 1 showing a flexible membrane extending from the access port and through the incision in tissue;

FIG. 5A is a bottom, perspective view of the access port of FIG. 1 shown being removed from the incision in tissue.

FIG. 5B is a top, perspective view of the access port of FIG. 1 shown being removed from the incision in tissue;

FIG. 6 is a front view illustrating a patient's skeletal structure with one embodiment of the presently disclosed surgical access assembly positioned within the intercostal space defined between adjacent ribs;

FIG. 7 is a bottom, perspective view of the body of an alternative embodiment of a surgical access port;

FIG. 8 is a top, perspective view of the body of FIG. 7;

FIG. 9 is a top, perspective view of the body of another alternative embodiment of a surgical access port;

FIG. 10 is a bottom, perspective view of the body of FIG. 9;

FIG. 11 is a top, perspective view of a body of another alternative embodiment of surgical access port; and

FIG. 12 is a bottom, perspective view of the body of FIG. 11.

### DETAILED DESCRIPTION

Various embodiments of the presently disclosed access assembly, or access port, and methods of using the same, will now be described in detail with reference to the drawings wherein like references numerals identify similar or identical elements. In the drawings, and in the following description, the term "proximal" should be understood as referring to the end of the access port, or component thereof, that is closer to the clinician during proper use, while the term "distal" should be understood as referring to the end that is further from the clinician, as is traditional and conventional in the art. Additionally, use of the term "tissue" hereinbelow should be understood to encompass both the patient's ribs, and any surrounding tissues. It should be also be understood that the term "minimally invasive procedure" is intended to include surgical procedures through small openings/incisions performed within a confined space such as the thoracic cavity or abdominal cavity.

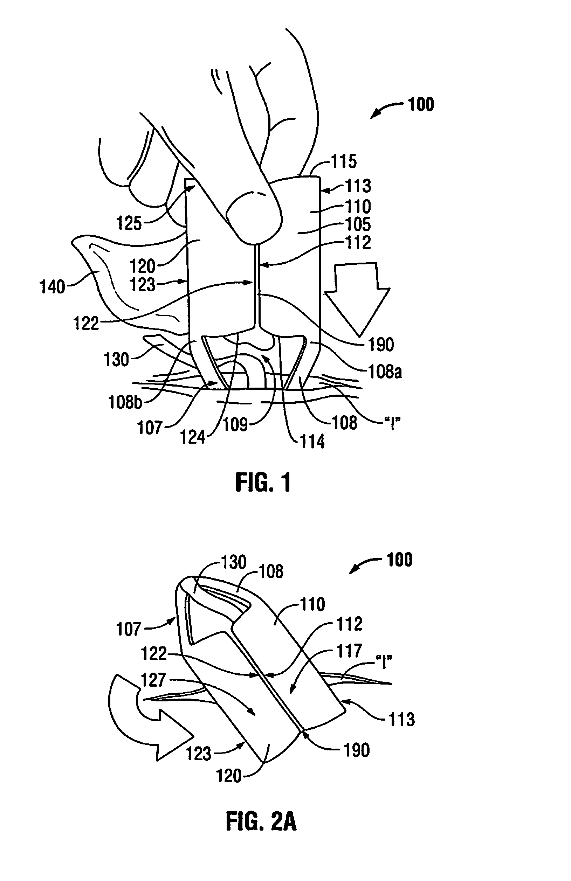

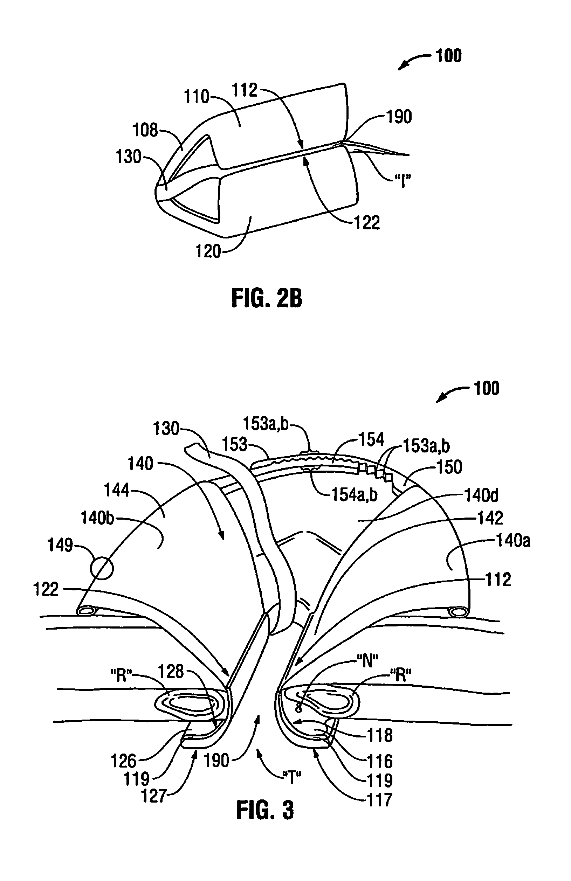

Referring now to FIGS. 1-5B, the presently disclosed surgical access port is shown generally identified by the reference numeral 100. In the embodiment of FIGS. 1-5B, the access port 100 is depicted as a thoracic port 100 that is configured and dimensioned for insertion into the intercostal space located between the adjacent ribs "R" (FIG. 3) of a patient in order to allow for the insertion and manipulation of one or more surgical instruments within the thoracic cavity. However, it is also envisioned that access port 100 may be configured and dimensioned to provide access to a variety of other internal body cavities and/or tissues. Further, access port 100 may be formed from any suitable biocompatible material of strength suitable for the purpose described herein, including, but not being limited to, polymeric materials.

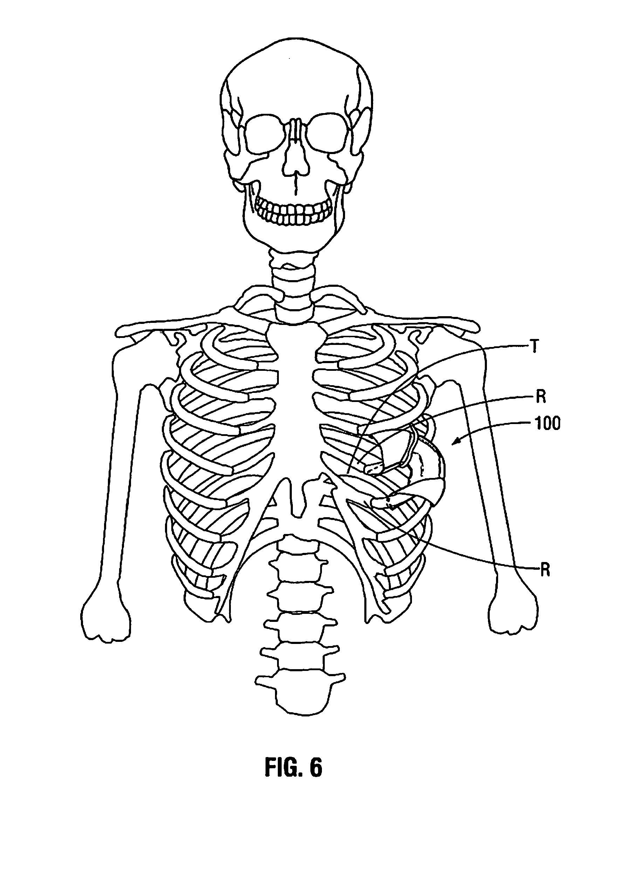

The access port 100 is configured and dimensioned to extend into a body cavity, e.g., the thoracic cavity "T" (FIGS. 3 and 6), through the intercostal space, and generally includes a body 105 having a substantially horseshoe shaped or substantially triangular shaped leading end 107 and first and second body members 110, 120 interconnected by the horseshoe shaped leading end 107. A ribbon 130 is attached to the horseshoe shaped leading end 107 to facilitate removal of the access port 100 from the cavity "T" and through incision "I" after the procedure. A flexible membrane 140 is attached at a distal end 142 thereof to opposed (inner) sides 112 and 122 of the first and second body members 110, 120, respectively, and is attached at a proximal end 144 to an adjustable ring 150. Access port 100 is moveable between a closed, or approximated position for insertion and removal, and an open, or spaced apart position wherein a passageway 190 (FIG. 3) extends therethrough to provide access to internal body cavities and/or tissue.

First and second body members 110, 120, include an outer side 113, 123, a leading end 114, 124 and a trailing end 115, 125, respectively. In the approximated, or closed position of access port 100, shown in FIG. 1, opposed sides 112, 122 of body members 110, 120, respectively, are positioned closer to each other, and preferably adjacent each other. End 108a of horseshoe shaped connector 108 extends from or is attached to leading end 114 of body member 110, and end 108b of horseshoe shaped connector 108 extends from or is attached to leading end 124 of body member 120. An opening 109 is defined between horseshoe shaped connector 108 and the leading ends 114, 124 of body members 110, 120, respectively.

Body members 110, 120 of access port 100 may be formed from a flexible or semi-rigid material to give access port 100 structural support while still allowing for some degree of flexibility. At least a portion of body members 110, 120 can be transparent to permit visualization through the access port 100 and into the surgical site. Body members 110, 120 may increase in thickness from their respective opposed sides 112, 122 to their respective outer sides 113, 123, as best shown in FIG. 3, and/or may include cushioning 119 (Fig. 3) disposed adjacent outer sides 113, 123 and extending along outwardly facing surfaces 116, 126 of body members 110, 120, respectively. This increased thickness and/or cushioning 119 helps protect surrounding tissue, e.g., ribs "R" and nerves "N," during the insertion and removal of surgical instrumentation and/or body tissue through the access port 100. As can be appreciated, the increased thickness of body members 110, 120 also allows the outer sides 113, 123 to be more rigid, or less flexible, than the opposed sides 112, 122 of body members 110, 120, respectively. As will become more apparent below, in a preferred embodiment, body members 110, 120 have increasing flexibility from the outer sides 113, 123 to the opposed sides 112, 122 such that the opposed sides 112, 122 may be moved apart from one another to create a passageway 190 extending through access port 100.

Access port 100 may be biased toward the approximated position wherein body members 110, 120 are positioned closer to and preferably adjacent one another. In this embodiment, if the body members 110, 120 are flexed to the open position and are not retained in the open position by a locking mechanism, body members 110, 120 would return under the bias to the approximated or closed position.

Each of the body members 110, 120 may define a similarly arcuate or curved profile on one or both surfaces, as viewed from either the leading ends 114, 124 or trailing ends 115, 125 of body members 110, 120, respectively. In other words, the outwardly facing surfaces 116, 126 of body members 110, 120, respectively, may define a generally convex configuration and/or the inwardly facing surfaces 117, 127 of body members 110, 120, respectively, may define a generally concave configuration. Accordingly, a saddle 118, 128 (FIG. 3) may be formed within each of the outwardly facing surfaces 116, 126 of body members 110, 120, respectively, of access port 100. As can be appreciated, saddles 118, 128 are relatively shallow when access port 100 is disposed in the approximated or closed position (FIG. 1). However, upon movement of access port 100 to the open, or spaced apart position (FIG. 3), saddles 118, 128 become more defined for seating ribs "R" therein. Correspondingly, as opposed sides 112, 122 are moved apart from one another, the outwardly facing surfaces 116, 126 of body portions 110, 120, respectively, become more convex, while the inwardly facing surfaces 117, 127 become more concave.

As best shown in FIG. 3, flexible member or membrane 140 is generally funnel shaped when tensioned and is coupled at distal end 142 thereof to opposed sides 112, 122 of body members 110, 120, respectively. More specifically, a first section 140a of flexible membrane 140 is mechanically coupled to opposed side 112 along the length of opposed side 112 of body member 110 and a second section 140b of flexible membrane is similarly mechanically coupled to opposed side 122 along the length of opposed side 122 of body member 120. A pair of end sections 140d of flexible membrane 140 connect the first and second sections 140a and 140b of flexible membrane 140 to one another, thereby defining the completed funnel shape, as shown in FIG. 3. In other words, flexible membrane 140 creates a funnel-shaped passageway 190 from the proximal end 144 thereof to the distal end 142 thereof. The funnel-shaped membrane 140 thus extends distally with the body members 110, 120 forming the distal- most portion of the funnel. As can be appreciated, the funnel is more conically shaped when body members 110, 120 are in the approximated position, i.e., where opposed sides 112, 122 of body members 110, 120 are adjacent one another, while the funnel is more cylindrically shaped when body members 110, 120 are in the open position, i.e., where opposed sides 112, 122 are spaced apart from one another.

It is envisioned that flexible membrane 140 is configured for soft tissue retraction. More particularly, it is envisioned that flexible membrane 140 has a sufficient elasticity to permit retraction of a wide range of tissue thicknesses since there may be a wide range of tissue thicknesses among different patients. It is also envisioned that flexible membrane 140 is of sufficient strength to properly retract body members 110, 120 when tensioned, to resist accidental puncture by sharp surgical instrumentation, and to resist tearing. Additionally, it is envisioned that flexible membrane 140 is made from a bio-compatible material to reduce the incidents of adverse reaction by a patient upon contact with the patient's tissue. The flexible membrane 140 can also be made of a transparent material to allow the user to better view the surgical site and surrounding tissue.

With continued reference to FIG. 3, the adjustable ring 150 is disposed at the proximal end 144 of flexible membrane 140. Adjustable ring 150 may be formed from a rigid biomaterial to define a structured opening to passageway 190 extending from the proximal end 144 of flexible membrane 140 through the body members 110, 120. More specifically, adjustable ring 150 may be disposed through a loop 149 formed at the proximal end 144 of flexible membrane 140. Proximal end 144 may be folded back onto and adhered to flexible membrane 140 to define loop 149 therebetween. Alternatively, adjustable ring 150 may be mechanically engaged with flexible membrane 140 in any other suitable configuration. In some embodiments, ring 150 can be flexible to conform to the contours of the patient's body.

Adjustable ring 150 includes structure to retain the ring in various positions. In the embodiment of FIG. 3, a ratcheting mechanism is provided with overlapping ends 153, 154, each defining a plurality of complementary teeth 153a, 154a, respectively, and notches 153b, 154b, respectively, on opposed surfaces thereof such that teeth 153a are engageable with notches 154b and teeth 154a are engageable with notches 153b to thereby expand or contract adjustable ring 150, as desired, and retain the ring in the select position. Accordingly, adjustable ring 150, and thus proximal end 144 of flexible membrane 140 disposed therearound, may define a minimum diameter wherein ends 153 and 154 of ring 150 are fully overlapping and wherein flexible membrane 140 is substantially un-tensioned, and a maximum diameter, wherein ends 153 and 154 of adjustable ring 150 are only slightly overlapping and wherein flexible membrane 140 is significantly tensioned. As will be described in more detail below, adjusting the ring diameter tensions and slackens the flexible membrane 140, thereby effecting opening and closing of the passageway 190 defined between body members 110, 120. It is also envisioned that any other suitable ratcheting, or adjustable member may be used to adjust adjustable ring 150 between a minimum and a maximum diameter. Further, the adjustable member 140 may include a locking mechanism to lock the flexible member 140 in a plurality of positions, e.g., defining a minimum diameter of ring 150, a maximum diameter of ring 150, and/or a plurality of intermediate diameters.

As mentioned above, the flexible membrane 140 is generally funnel-shaped when tensioned and extends distally and inwardly from the adjustable ring 150, which is disposed at the proximal end 144 of flexible membrane 140, ultimately attaching at a distal end 142 thereof to the body members 110, 120. Moreover, the first and second sections 140a, 140b and end sections 140d of flexible membrane 140 may be integral with one another, i.e., formed as a single membrane, or may be formed as separate sections engaged with one another via conventional means. It is envisioned that distal end 142 of flexible membrane 140 be sealingly attached, or integral with body members 110, 120, such that the passageway 190 extending through access port 100 is isolated from tissue surrounding the incision "I." In a preferred embodiment flexible membrane 140 and body members 110, 120 completely shield the incision "I," to reduce the risk of tissue damage and/or infection during the surgical procedure.

With reference now to FIGS. 2A-2B, horseshoe shaped connector 108 extends from leading ends 114, 124 of body members 110, 120, respectively, of access port 100. Horseshoe shaped connector 108 may be formed integrally with or may be attached to leading ends 114, 124 via suitable means. Horseshoe shaped connector 108 be made from a strong, rigid material to maintain a fixed spatial relation between body members 110, 120. To this end, horseshoe shaped connector 108 may be reinforced to provide further structural support thereto. Horseshoe shaped connector 108 may be configured to maintain outer sides 113, 123 of body members 110, 120, respectively, relatively fixed with respect to one another, while opposed inner sides 112, 122 are flexible with respect to outer sides 113, 123, thereby flexing body members 110, 120. Thus, the passageway 190 extending through access port 100 is expandable between a minimum width, wherein opposed sides 112, 122 of body members 110, 120 are adjacent one another, and a maximum width wherein opposed sides 112, 122 of body members 110, 120 are flexed apart from one another and with respect to the outer sides 113, 123 of body members 110, 120, respectively. As can be appreciated, in the illustrated embodiment, the maximum width of passageway 190 does not exceed the distance between outer sides 113, 123, which are maintained in fixed relation relative to one another by horseshoe shaped connector 108.

A second horseshoe shaped connector (not shown), substantially similar to horseshoe shaped connector 108 may be disposed on the trailing ends 115, 125 of body members 110, 120, respectively, to provide further structural support to body members 110, 120, and more specifically, to outer sides 113, 123 of body members 110, 120, respectively.

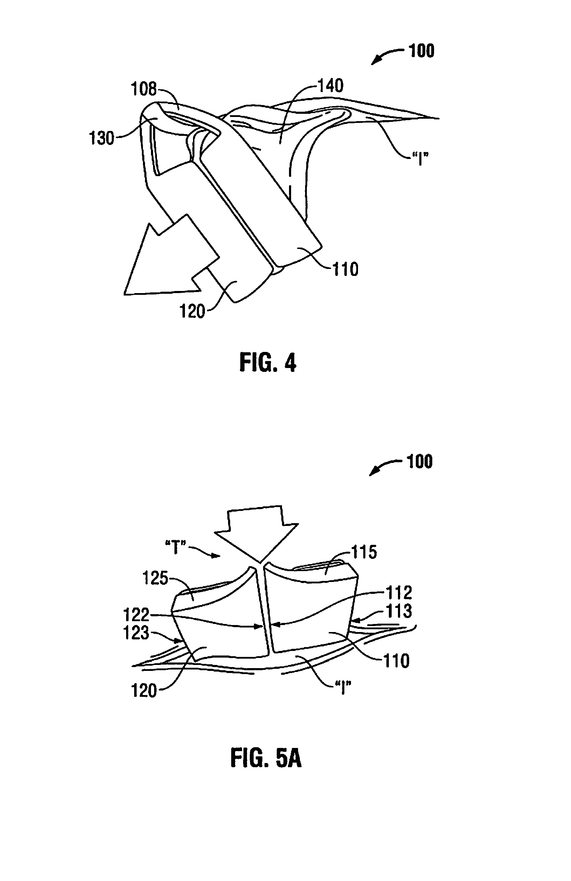

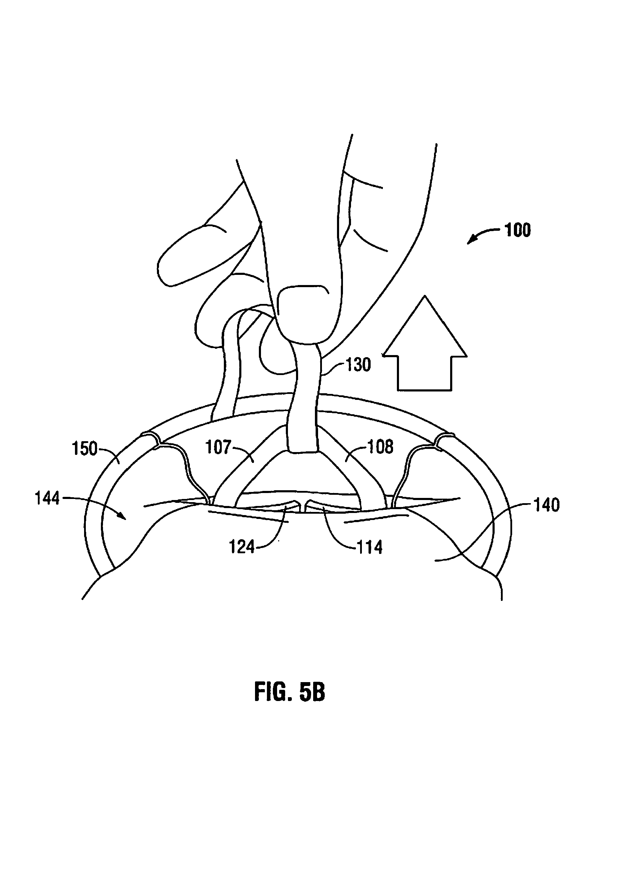

Ribbon 130, as best shown in FIGS. 4 and 5B, is disposed about horseshoe shaped connector 108 and extends therefrom. Ribbon 130 may be adhered to, looped around, or otherwise engaged with horseshoe shaped connector 108. Ribbon 130 has sufficient length to extend proximally from access port 100 out through the incision "I" to be grasped by the user. As will be described in more detail below, ribbon 130 is configured for removal of access port 100 from the incision "I." In some embodiments, ribbon 130 can be provided to facilitate manipulation of access port 100 during the insertion and use of the access port 100. It is envisioned that more than one ribbon 130 may be provided, to further facilitate manipulation of access port 100. Alternatively, or in conjunction with ribbon 130, flexible membrane 140 may be used to manipulate, orient, or position access port 100.

The use and operation of the access port 100 will be now discussed during the course of a minimally invasive thoracic procedure by way of example. As will be appreciated in view of the following, access port 100 is easily inserted, manipulated, and removed from a patient's body. Further, the access port 100 is minimally intrusive, flexible to conform to a patient's anatomy, and provides good visibility into the thoracic cavity "T" (FIG. 3). Additionally, the funnel-shaped, low-profile configuration of access port 100 is particularly advantageous, for example, in the removal, or retrieval, of tissue specimens from within the body.

Initially, an opening, or incision "I," is made in the patient's outer tissue wall of the thoracic body cavity by conventional means. The incision "I" is made between adjacent ribs "R," extending along the intercostal space. In other words, a relatively narrow, elongated incision "I" is made between adjacent ribs "R."

In preparation for insertion through the incision "I," access port 100 is rotated to a vertical position shown in FIG. 1, wherein the horseshoe shaped leading end 107 is distal, or closer to the incision "I," and wherein the trailing ends 115, 125 of body members 110, 120 are proximal, or closer to the user. At this point, the body members 110, 120 are biased in the approximated position, preferably biased in this position, such that access port 100 is relatively thin and the passageway 190 therethrough defines a minimum width, as described above, or is closed if sides 112, 122 are in abutment as in some embodiments. This alignment of the access port 100 with the incision "I" allows access port 100 to be inserted through the narrow incision "I" between the adjacent ribs "R" with limited, if any, expansion of the incision and minimal trauma to surrounding tissue. Ribbon 130 extends from horseshoe shaped connector 108 away from the incision "I" such that a portion of ribbon 130 extends from the incision "I," as shown in FIG. 1.

As shown in FIG. 1, the user then grasps the access port 100, e.g., with his/her fingers or with any other suitable surgical tool, and advances the access port 100 distally through the incision "I," led by horseshoe shaped leading end 107. It is envisioned that the leading and trailing ends 114, 124 and 115, 125 of body members 110, 120, respectively, may define a curved configuration to decrease the likelihood of access port 100 "catching" on tissue during insertion and removal of access port 100 from the incision "I." Horseshoe shaped leading end 107 and body members 110, 120 are fully inserted into incision "I," while flexible membrane 140 extends proximally from incision "I."

Once the body members 110, 120 of access port 100 are fully disposed through the incision "I," as shown in FIG. 2A, membrane 140 may be pulled proximally to align the access port 100 for deployment. More specifically, after insertion of access port 100, as can be appreciated, horseshoe shaped leading end 107 is positioned furthest into the body cavity, while trailing ends 115, 125 of body members 110, 120, respectively are closest to the incision "I," i.e., access port 100 is oriented as shown in FIG. 1. With access port 100 fully disposed within the internal body cavity, membrane 140 may be pulled, causing horseshoe shaped leading end 107 to be pulled back towards the incision "I," thereby rotating access port 100. Membrane 140 is pulled until body members 110, 120 of access port 100 are positioned substantially parallel to the surface of tissue through which incision "I" has been made, as shown in FIG. 2A. Lateral translation of membrane 140 may then be effected such that opposed sides 112, 122 of body members 110, 120, respectively, align substantially with the opposing sides of the incision "I" and such that the passageway 190 defined between opposed sides 112, 122 of the body members 110, 120, respectively, aligns with the incision "I," as shown in FIG. 2B. More particularly, the outer sides 113, 123 of body members 110, 120 are positioned adjacent to and distal of the ribs "R," while opposed sides 112, 122, defining passageway 190 therebetween, are positioned adjacent and distal of the incision "I." As mentioned above, multiple ribbons 130 may be provided on horseshoe shaped leading end 107 or at other positions on access port 100 to facilitate removal of access port 100 after completion of the procedure.

It should be noted that, as shown in FIG. 2B, when access port 100 is inserted and positioned within incision "I," access port 100 is oriented such that the concave, outwardly facing surfaces 116, 126 of body members 110, 120 are facing proximally (toward the incision "I") and such that the convex, inwardly facing surfaces 117, 127 of body members 110, 120 are facing distally (toward the thoracic body cavity "T"). As can be appreciated, in this orientation, the opposed sides 112, 122 of body members 110, 120, respectively, extend proximally at least partially toward the incision "I" due to the curved surfaces of body members 110, 120. Flexible membrane 140 extends proximally from opposed sides 112, 122 of body members 110, 120, respectively. More specifically, and although not viewable in FIGS. 2A-2B, flexible membrane 140, having adjustable ring 150 disposed at a proximal end thereof, extends from opposed sides 112, 122 of body members 110, 120 proximally through the incision "I." Ring 150 is positioned adjacent an external surface of tissue and is initially disposed in the minimum, un-tensioned configuration, i.e., wherein ends 153, 154 are substantially overlapping to form a minimum diameter of ring 150. The positioning of ring 150 adjacent the external surface of tissue provides a desirable low-profile configuration that allows for greater maneuverability of surgical instrumentation within access port 100.

From the position described above and shown in FIG. 2B, access port 100 may be expanded from the approximated position to the open (spread) position to provide access to an internal body cavity, e.g., the thoracic cavity "T" (FIGS. 3 and 6). In order to expand the access port 100 from the approximated position to the open position, adjustable ring 150 is ratcheted, or expanded, from its minimum diameter to a larger diameter. As can be appreciated, as ring 150 is expanded, ring 150 is moved along the external surface of tissue radially away from incision "I," thereby tensioning flexible membrane 140 and pulling flexible membrane 140 proximally through the incision "I," eventually pulling flexible membrane 140 radially outwardly from the incision "I" along the external surface of tissue. As flexible membrane 140 is tensioned and pulled proximally through the incision "I," opposed sides 112 and 122 of body members 110, 120, respectively, are pulled proximally through the incision "I" until flexible membrane 140 is no longer disposed through incision "I" but, rather, completely extends along the external surface of tissue. Body members 110, 120 are thus disposed through the incision "I" with opposed sides 112, 122 extending toward a proximal end of incision "I" and with outer sides 113, 123 extending toward a distal end of incision "I," as shown in FIG. 3. The increased flexibility of body members 110, 120 from outer ends 113, 123 to opposing ends 112, 122 allows body members 110, 120 to be flexed in response to the tensioning and pulling of flexible membrane 140.

Moreover, horseshoe shaped connector 108 helps maintain outer sides 113, 123 in position adjacent and distal of ribs "R." In other words, outer sides 113, 123 are retained within the thoracic cavity "T," distal of the ribs "R," while opposed sides 112, 122 are flexed proximally and apart from one another through the incision "I" in response to the pulling of flexible membrane 140 by the expansion of the adjustable ring 150. Further, it is envisioned that grips (not explicitly shown) may be disposed on the outwardly facing surfaces 116, 126 and, more particularly, lining the saddles 118, 128 of body members 110, 120, respectively, to anchor the body members 110, 120 in position and to prevent slippage.

As shown in FIG. 3, as adjustable ring 150 is moved toward a maximum diameter, outwardly facing surfaces 116, 126 of body members 110, 120 engage tissue adjacent ribs "R" within saddles 118, 128 and retract the tissue to expand the incision. (In some embodiments, the ribs "R" can be urged apart from one another to spread the ribs to expand the intercostal space). Further, as can be appreciated, as opposed sides 112, 122 of body members 110, 120 are flexed proximally and outwardly from one another to expand tissue adjacent ribs "R," the passageway 190 defined through access port 100 is expanded from the approximated position defining a minimum width to an open position, wherein the passageway 190 defines a larger width, as best shown in FIG. 3. The locking mechanism, e.g., interlocking teeth 153a, 154a and notches 153b, 154b of ends 153, 154 of ring 150, allows access port 100 to be retained in the open position (FIG. 3). Further, the interlocking teeth 153a, 154a and notches 153b, 154b of ring 150 allow for locking of access port 100 in a plurality of intermediate positions between the approximated position and the spread or open position. Such a feature accommodates different anatomies of different patients, i.e., their intercostal spacing may be different, and accounts for the desirability in some procedures to urge the ribs "R" apart further, while in other procedures to simply provide access to the internal cavity without increasing the spacing between the adjacent ribs "R."

Once access port 100 is retained or locked in the open position as described above, surgical instrumentation may be inserted through passageway 190 to perform the surgical procedure therein. As shown in FIG. 3, body members 110, 120 maintain passageway 190 while protecting the incision "I" and the surrounding tissue. Ribs "R" and nerves "N" are protected within saddles 118, 128 by the thickened portions of body members 110, 120 and/or the additional cushioning 119. Flexible membrane 140 extends radially outwardly from incision "I" and protects the external surface of tissue, while adjustable ring 150 maintains access port 100 in the open position. Thus, the incision "I" and surrounding tissue is protected while providing access to the thoracic cavity "T" with minimal pain to the patient and minimal tissue damage. Additionally, as mentioned above, the low-profile configuration of flexible membrane 140 and ring 150 allows for greater access to the thoracic cavity "T," and for greater manipulation of instrumentation disposed through passageway 190

The inwardly facing surfaces 117, 127 of the body members 110, 120, respectively, may be coated with a lubricant, or gel, to aid in the insertion and removal of surgical instrumentation and/or tissue specimen from access port 100.

A textured surface can optionally be placed on the outer (contact) surfaces 116, 126 to increase the grip on the intercostal tissue. The membrane 140 can also optionally have a textured surface to enhance gripping of tissue.

Upon completion of the surgical procedure, adjustable ring 150 is collapsed or "unlocked" and returned to the minimum diameter, thereby un-tensioning flexible membrane 140 and allowing body members 110, 120 to return under the bias to the approximated, or closed position shown in FIG. 2B, and allowing the tissue adjacent ribs "R" to return to its initial position (and in some embodiments the ribs "R" to contract back to their at-rest position). As body members 110, 120 are returned to the un-flexed, closed position, access port 100 returns to the thin, relatively flat shape of the approximated position. In this approximated position, access port 100 may be easily removed from the incision "I." More specifically, ribbon 130 may be pulled proximally, thereby pulling horseshoe shaped leading end 107 of access port 100 proximally and rotating access port 100 into a removal position, as best shown in FIG. 4. Upon further translation of ribbon 130, as shown in FIGS. 5A-5B, access port 100, lead by horseshoe shaped leading end 107 is translated proximally through the incision "I" until the access port 100 has been completely removed form the incision "I." Finally, the incision "I" may be closed off, e.g., sutured closed.

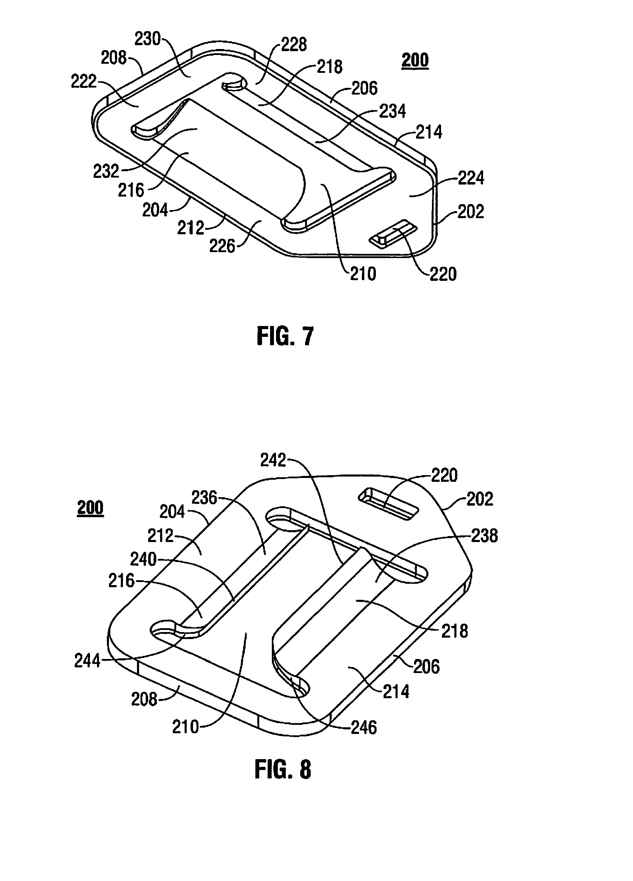

Referring to FIGS. 7 and 8, there is disclosed an alternative body 200 to that of body 105 described hereinabove, for use in surgical access port 100. Body 200 generally includes a triangular or horseshoe shaped substantially rigid leading end 202, first and second body members 204 and 206 and a substantially rigid connecting trailing end 208. Horseshoe shaped leading end 202, first and second body members 204 and 206 and trailing end 208 define a passageway 210 there between for receipt of a flexible member, such as, for example flexible membrane 140 (FIG. 3) described hereinabove. First and second body members 204 and 206 include respective first and second rigid side walls 212 and 214 having first and second flexible, inwardly directed wings 216 and 218 extending therefrom. A ribbon port 220 is provided in leading end 202 for receipt of a ribbon, such as, for example ribbon 130 (FIG. 2A) described hereinabove.

Rigid leading end 202, first and second rigid side walls 212 and 214 and rigid trailing end 208 may be formed from separable or separate components or may, as shown, be formed as an integral structure and formed from a variety of biocompatible rigid materials such as, for example, polymers, metals, ceramics, etc. In order to secure flexible membrane 140 to body 200, body 200 is provided with a membrane bonding surface 222 on the undersides 224, 226, 228 and 230 of leading end 202, first rigid side wall 212, second rigid side wall 214 and rigid trailing end 208, respectively. Membrane bonding surface 222 may be provided as an adhered sheet of material or as a coating on the surfaces. Membrane bonding surface 222 is provided to supply a surface to which distal end 142 (FIG. 3) of flexible membrane 140 can be affixed. The bonding surfaces enable 360 degree membrane attachment.

First and second flexible wings 216 and 218 have respective inward facing surfaces 232 and 234 (FIG. 7) and respective concave outward facing surfaces 236 and 238 (FIG.8). As shown, first and second wing members 216, 218 are positioned on opposing sides of passageway 210 and extend along a length of the body 200. (The length of the body 200 in the illustrated embodiment exceeding its width). First and second flexible wing members 216 and 218 can be transparent to permit visualization through axis port 100 described herein above. Additionally, first and second flexible wings 216 and 218 in the illustrated embodiment increase in thickness from their respective opposed sides 240 and 242 to their respective outer sides 244 and 246. Similar to body 105 described herein above, flexible wings 216 and 218 may include cushioning (not shown) adjacent concave outward facing surfaces 236 and 238. The increased thickness and/or cushioning helps protect surrounding tissues, and e.g., ribs "R" and it nerves "N" (FIG. 3) during insertion and removal of surgical instrumentation and/or body tissue through axis port 100. The increased thickness of flexible wings 216 and 218 also allows respective outer sides 244 and 246 to be more rigid, or less flexible, then opposed sides 240 and 242 to accommodate a variety of intercostal spaces. Alternatively, or additionally, the wings can be made of a material of varying thickness to provide less flexible outer sides.

The use of body 200 in surgical access port 100 will now be described. Initially, distal end 142 of flexible membrane 140 (FIG. 3) is affixed to membrane bonding surface 222 on body 200 (FIG. 7). Adjustable ring 150 described hereinabove, (FIG. 3) may be provided to maintain proximal end 144 of flexible membrane 140 in an open condition to receive surgical instruments. However, it should be noted that in some embodiments adjustable ring 150 is not needed to maintain passageway 210 through body 200, with body 200 forming a relatively rigid and completely circumferential outer periphery around passageway 210. A ribbon similar to ribbon 130 (FIG. 2B) is affixed through ribbon port 220.

In use, surgical access port 100, incorporating body 200, functions similar to that described hereinabove. Leading end 202 of body 200 is initially inserted through incision "I" (FIG. 1) until body 200 has passed completely therethrough. Note that although the flexible wings 204, 206 are shown extending upwardly in the insertion position, it is also contemplated that the wings are hinged at a base to provide a more planar insertion profile.

Thereafter, body 200 is rotated (FIG. 2A) to bring concave outward facing surfaces 236 and 238 of flexible wings 204 and 206 into engagement with ribs "R" (FIG. 3). Then, the outer ring 150 is expanded to a larger diameter as described in detail above to expand the access port 100 and provide tissue retraction. Thereafter, a thoracic surgical procedure may be performed by the insertion and operation of surgical instrumentation (not shown) through passageway 210 of body 200. Once a surgical procedure has been completed, the adjustable outer ring 150 is returned to the smaller diameter, untensioning the membrane, and body 200 may then be removed in a manner similar to that described hereinabove with regard to body 105 by manipulation of ribbon 130 (FIG. 5).

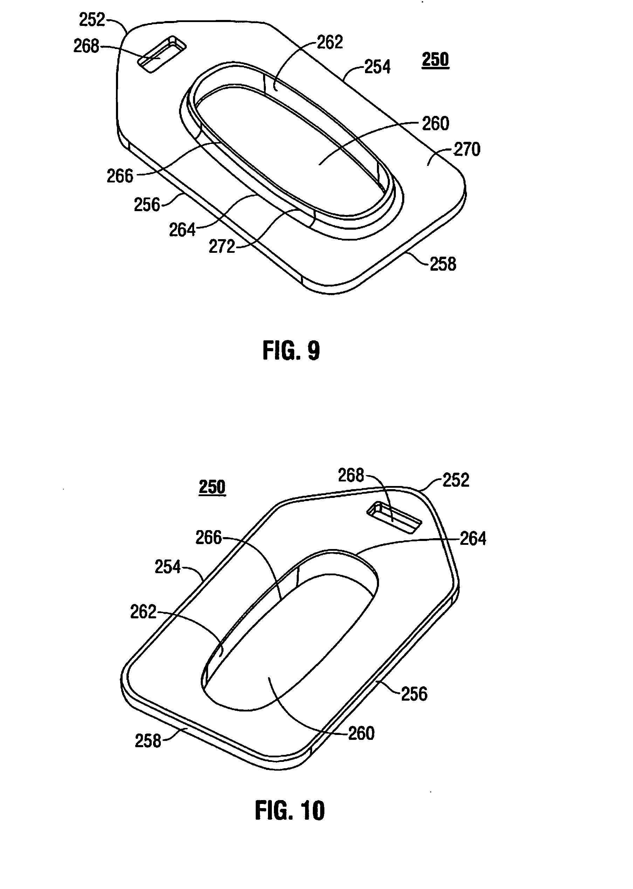

Referring now to FIGS. 9 and 10, there is disclosed an alternative body 250 for use with surgical access port 100 described herein above. Body 250 generally includes a triangular or horseshoe shaped leading end 252 and respective first and second body members 254 and 256. A connecting or trailing end 258 is provided to connect first and second body members 254 and 256. Leading end 252, first and second body members 254 and 256 and trailing end 258 form a substantially rigid substrate and define a passageway 260 through body 250 for receipt of surgical instrumentation. In the illustrated embodiment, passageway 260 is substantially oval. A circumferential flexible wall 262 surrounds and extends from passageway 260 and is affixed to leading end 252, first and second body members 254 and 256 and trailing end 258 at first or connecting end 264 of flexible wall 262. Alternatively, flexible wall 262 may be formed integrally with leading end 252, first and second body members 254 and 256 and trailing end 258. Flexible wall 262 includes a second or free end 266. A ribbon port 268 is provided in leading end 252 for receipt of a ribbon such as, for example, ribbon 130 described hereinabove. Leading end 252, trailing end 258 and first and second body members 254, 256 can be formed from separate or separable components, or as shown formed from an integral structure.

Flexible wall 262 may be transparent to facilitate visualization therethrough. Additionally, flexible wall 262 may increase in thickness from free end 266 to connecting end 264. In use, flexible wall 262 extends proximally toward the incision. A cushioning or relatively soft material 270 may be provided on body 250 to cushion the engagement of body 250 with ribs "R" and surrounding tissue as in FIG. 3. The cushioning surface can have a cutout along an outer edge to allow it to fold flatter during insertion into the patient. The cushioning can also have a tapered or funnel-like internal profile to facilitate specimen removal from the body cavity.

Surgical access port 100, incorporating body 250, is assembled in a manner substantially identical to that described hereinabove with regard to body 200. Specifically, a distal end 142 of flexible membrane 140 (FIG. 3) is secured to the substantially rigid substrate formed by a leading end 252, first and second body members 254 and 256 and trailing end 258. Flexible membrane 140 passes around flexible wall 262 and back through passageway 260. Alternatively, the membrane 140 can be attached to flexible wall 262. Ribbon 130 is affixed to ribbon port 268 formed in leading end 252.

In use, leading end 252 is inserted through incision "I" as in the port of FIG. 1 and body 250 is rotated into position (FIG. 2A) such that an outer edge 272 of flexible wall 262, along with soft material 270, engages ribs "R" (see FIG. 3). After expansion of the adjustable ring 150 as described above, surgical instrumentation may be inserted through passageway 260 and a surgical operation performed. Once the surgical operation has been completed, ribbon 130 may be pulled and manipulated to extract body 250 through incision "I". In this manner, body 250, incorporated in surgical access port 100, provides both a rigid support about an instrument passageway as well as a fully circumferential wall 262 which protects the entire incision "I" from engagement with surgical instrumentation. It also provides a 360 degree membrane.

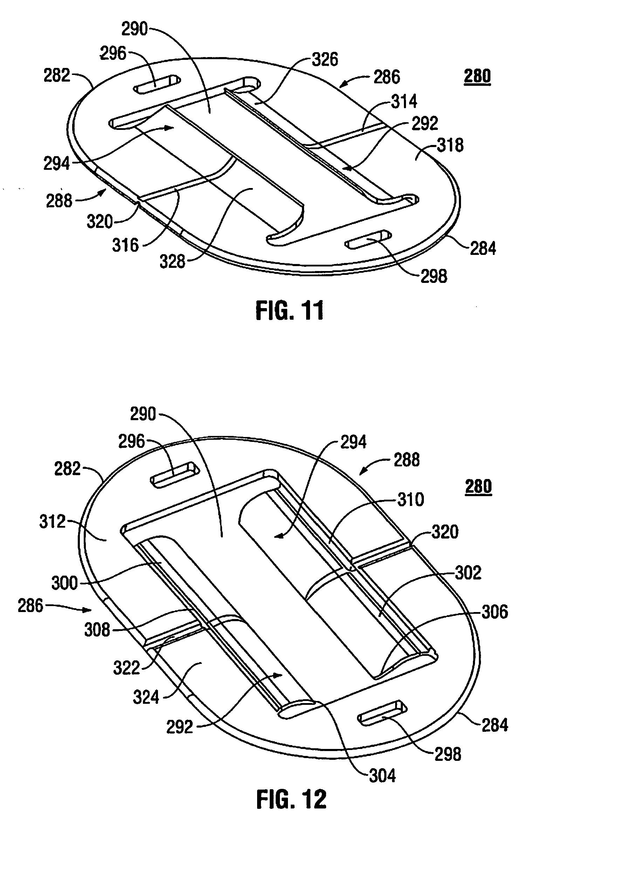

Referring now to FIGS. 11 and 12, there is disclosed a further alternative body 280 for use in surgical access port 100 described hereinabove. Body 280 is symmetrical and generally includes a first or leading end 282 and a second or trailing end 284. First and second body members 286 and 288 extend between leading end 282 and trailing end 284. Leading end 282, trailing end 284 and first and second body members 286 and 288 define a passageway 290 therethrough for receipt of surgical instrumentation. A first flexible wing member 292 extends from first body member 286 and into passageway 290. Similarly, a second flexible wing member 294 extends from second body member 288 into passageway 290. First and second flexible wing members 292, 294 are positioned on opposing sides of passageway 290 and extend along a length of the body 280. (The length of the body 280 in the illustrated embodiment exceeding its width). In order to facilitate removal of body 280 through an incision, first and second ends 282 and 284 are provided with respective first and second ribbon ports 296 and 298. First and second ribbon ports 296 and 298 are provided to receive ribbons 130 in the manner described hereinabove, however in this version a ribbon can be attached to both ends so the body 250 can be pulled from either end for removal. Use of a single ribbon as in the embodiment of FIG. 1 is also contemplated.

With specific reference to FIG. 12, first and second flexible wings 292 and 294 preferably decrease in thickness from first and second attachment ends 300 and 302 to first and second free ends 304 and 306. Additionally, first and second flexible wings 292 and 294 may be transparent in order to facilitate visualization of a surgical procedure. In order to allow first and second flexible wings 292 and 294 to fold flat during insertion, first flexible wing 292 is connected to first body member 286 along a first wing fold 308. Likewise, second flexible wing 294 is connected to second body member 288 along a second wing fold 310. First and second wing folds 308 and 310 are formed in an undersurface 312 of body 280.

While the prior disclosed bodies are configured for initial insertion through an incision at a leading end, and body 280 can likewise be inserted in this way, body 280 is also configured to be folded in half for insertion through an incision. First and second central folds 314 and 316 extend partially through an upper surface 318 of body 280 (FIG. 11). Specifically, first and second central folds 314 and 316 extend partially through first and second body members 286 and 288. With reference to FIG. 12, third and fourth central folds 320 and 322 extend partially through undersurface 312 of body 280. First and second central folds 314 and 316 along with third and fourth central folds 320 and 322 allow body 280 to be folded in half thereby allowing body 280 to be inserted through an incision with either leading and trailing ends 282 and 284 inserted first or first and second central folds 314 and 316 inserted first or third or fourth central folds 320 and 322 inserted first into the incision. As shown, fourth central fold 322 bisects first wing fold 308 and third central fold 320 bisects second wing fold 310.

Body 280 is assembled into surgical access port 100 in a manner similar to that described hereinabove. Specifically, distal end 142 of flexible membrane 140 is affixed to a membrane binding surface 324 provided on undersurface 312. Flexible membrane 140 may be provided with ring 150 to maintain proximal 144 of flexible membrane 140 in an open condition. Flexible membrane 140 passes through passageway 290 and extends 360 degrees. A pair of ribbons 130 (FIG. 2B) are affixed through first and second ribbon ports 296 and 298 to aid removal from either end.

In use, body 280 is inserted through incision "I" (FIG. 1). As noted herein above, body 280 can be inserted through incision "I" with first or leading end 282 initially inserted through incision "I" or with second or trailing end 284 initially inserted through incision "I". Alternatively, body 280 may be folded in half along first, second, third and fourth central fold lines 314, 316, 320 and 322, respectively, and then inserted through incision "I" such that body 280 is inserted with central fold lines 314, 316, 320 and 322 initially inserted through incision "I". Thereafter, similar to the procedures described herein above, body 280 may be rotated into position (FIG. 2A) such that first and second outward facing surfaces 326 and 328 (FIG. 11) engage ribs "R" (FIG. 3).

After expansion of the adjustable outer ring 150 in the manner described above to retract tissue, a surgical procedure can then be performed by insertion of surgical instrumentation through flexible membrane 140 and passageway 290 defined through body 280. Once a surgical procedure has been completed, one or both ribbons 130 may be manipulated to extract body 280 back through incision "I". In this manner, body 280 provides a rigid perimeter about passageway 290 for receipt of surgical instruments therethrough. Additionally, the multiple folds provided in body 280 allow body 280 to be inserted through a surgical incision in a variety of manners depending upon surgical necessity.

Although described for use in thoracic procedures, it should also be understood that the access ports described herein can be used in other minimally invasive surgical procedures.

Persons skilled in the art will understand that the devices and methods specifically described herein and illustrated in the accompanying figures are non-limiting exemplary embodiments, and that the description, disclosure, and figures should be construed merely exemplary of particular embodiments. It is to be understood, therefore, that the present disclosure is not limited to the precise embodiments described, and that various other changes and modifications may be effected by one skilled in the art without departing from the scope or spirit of the disclosure. Additionally, it is envisioned that the elements and features illustrated or described in connection with one exemplary embodiment may be combined with the elements and features of another without departing from the scope of the present disclosure, and that such modifications and variations are also intended to be included within the scope of the present disclosure. Accordingly, the subject matter of the present disclosure is not to be limited by what has been particularly shown and described, except as indicated by the appended claims.

The present invention may also be described by reference to the following numbered paragraphs:-
1. A surgical access assembly for positioning within an opening in tissue, the surgical access assembly comprising: a body having a leading end, a trailing end and first and second body members extending between the leading end and the trailing end, the leading end, trailing end and first and second body members defining a passageway therethrough; first and second flexible wing members extending proximally from the body; and a flexible member attached to the body and extending proximally therefrom and surrounding the passageway.
2. The surgical access assembly as recited in paragraph 1, wherein a distal end of the flexible member is affixed to a member bonding surface on the body.
3. The surgical access assembly as recited in paragraph 1, wherein at least a portion of the member extends through the passageway.
4. The body as recited in paragraph 1, wherein the flexible member is attached to the flexible wing members.
5. The body as recited in paragraph 1, wherein the flexible wing members each have a free end, the free end of each flexible wing member movable from a first position to a second position where the free ends are spaced further apart.
6. The body as recited in paragraph 1, wherein the leading end has a substantially triangular shape.
7. The body as recited in paragraph 1, wherein at least one of the leading end and trailing end has a ribbon port formed therethrough.
8. The body as recited in paragraph 1, wherein each of the leading end and trailing end has a ribbon port formed therethrough.
9. The body as recited in paragraph 1, wherein the first and second flexible wing members extend into the passageway.
10. The body as recited in paragraph 1, wherein each of the first and second flexible wing members has concave outward facing surfaces for engagement with tissue adjacent ribs of a patient.
11. The body as recited in paragraph 1, wherein each of the first and second flexible wing members decreases in thickness from the first and second body members to first and second free ends of the first and second flexible wing members.
12. The body as recited in paragraph 1, wherein the first and second body members include first and second central fold lines formed in the first and second body members.
13. The body as recited in paragraph 12, wherein the first and second central fold lines are formed in upper surfaces of the first and second body members.
14. The body as recited in paragraph 13, wherein second and third central fold lines are formed in under surfaces of the first and second body members.
15. The body as recited in paragraph 12, wherein the first flexible wing member is connected to the first body member along a first wing fold.
16. The body as recited in paragraph 15, wherein the first central fold line bisects the first wing fold.
17. The body as recited in paragraph 15, wherein the second flexible wing member is connected to the second body member along a second wing fold.
18. The body as recited in paragraph 17, wherein the second fold line bisects the second wing fold.
19. The body as recited in paragraph 1, wherein the first and second flexible wing members are on opposing sides of the passageway.
20. The body as recited in paragraph 19, wherein the body has a length greater than a width, and the flexible wing members are oriented along the length of the body

## Claims

1. A surgical access assembly for positioning within an opening in tissue, the surgical access assembly comprising:
a body having a leading end, a trailing end and first and second body members extending between the leading end and the trailing end, the leading end, trailing end and first and second body members defining a passageway therethrough;
first and second flexible wing members extending proximally from the body; and
a flexible member attached to the body and extending proximally therefrom and surrounding the passageway.

2. The surgical access assembly as recited in claim 1, wherein a distal end of the flexible member is affixed to a flexible member bonding surface on the body.

3. The surgical access assembly as recited in claims 1 or 2, wherein at least a portion of the flexible member extends through the passageway.

4. The body as recited in claims 1, 2 or 3, wherein the flexible member is attached to the flexible wing members.

5. The body as recited in any of claims 1-4, wherein the flexible wing members each have a free end, the free end of each flexible wing member movable from a first position to a second position where the free ends are spaced further apart.

6. The body as recited in any of claims 1-5, wherein the leading end has a substantially triangular shape.

7. The body as recited in any of claims 1-6, wherein at least one of the leading end and trailing end has a ribbon port formed therethrough.

8. The body as recited in any of claims 1-7, wherein the first and second flexible wing members extend into the passageway.

9. The body as recited in any of claims 1-8, wherein each of the first and second flexible wing members has concave outward facing surfaces for engagement with tissue adjacent ribs of a patient.

10. The body as recited in any of claims 1-9, wherein each of the first and second flexible wing members decreases in thickness from the first and second body members to first and second free ends of the first and second flexible wing members.

11. The body as recited in any of claims 1-10, wherein the first and second body members include first and second fold lines formed in upper surfaces of the first and second body members and second and third central fold lines formed in under surfaces of the first and second body members.

12. The body as recited in any of claims 1-11, wherein the first flexible wing member is connected to the first body member along a first wing fold and the second flexible wing member is connected to the second body member along a second wing fold.

13. The body as recited in claim 12, wherein the first fold line bisects the first wing fold and the second fold line bisects the second wing fold.

14. The body as recited in any of claims 1-13, wherein the first and second flexible wing members are on opposing sides of the passageway.

15. The body as recited in any of claims 1-14, wherein the body has a length greater than a width, and the flexible wing members are oriented along the length of the body.
